# EUROPEAN PATENT APPLICATION

(11) **EP 4 575 000 A1**
(43) Date of publication of application: **25.06.2025**
(21) Application number: 24219459.5
(22) Date of filing: 12.12.2024
(51) Int. Cl.: C12Q 1/6886

(54) **METHOD OF ANALYSIS AND CLASSIFICATION OF BIOLOGICAL MATERIAL FOR OVARIAN CANCER DETECTION AND PANEL OF RT-QPCR PROBES**

(30) Priority: 21.12.2023 PL 44721423
(71) Applicant: Gdanski Uniwersytet Medyczny, 80-210 Gdansk (PL)
(72) Inventor: Supernat, Anna, Gdynia (PL); Zaczek, Anna, Gdynia (PL); Pastuszak, Krzysztof, Elbl g (PL); Sieczczynski, Micha, Dzia dowo (PL)
(74) Representative: JWP Patent & Trademark Attorneys

(57) **Abstract**

The subject of the invention is a method for analyzing and classifying a sample of biological material to detect ovarian cancer in an individual using the RT-qPCR method, as well as an RT-qPCR probe panel configured to perform the steps of this method.

## Description

The subject of the invention is a method for analyzing and classifying biological material for the detection of ovarian cancer, as well as a panel of molecular probes for the RT-qPCR platform (Reverse Transcription quantitative Polymerase Chain Reaction) configured to perform the stages of the mentioned method.

Routine diagnostics, which is currently in use, often result in the detection of cancer at an advanced stage (III or IV), when the disease process has spread beyond its initial site, affecting lymph nodes or even forming distant metastases. A cancer that has progressed to this stage offers no hope for complete recovery. Patients with ovarian cancer, despite an initially good response to first-line treatment, eventually experience increasingly frequent recurrences of the disease. Next, resistance to treatment develops or therapy can no longer be continued due to poor health, leading to death.

The challenge for patients with undetected ovarian cancer is the lack of a means to obtain a quick and accurate diagnosis. A test with greater sensitivity, specificity, and dynamism is needed, as the routinely used CA-125 marker measurement is nonspecific and has low sensitivity. The CA-125 marker level can remain low even in the advanced stages of the cancer. Additionally, the half-life of this protein in the blood is significantly longer than that of, for example, transcriptionally altered platelets. This means that a patient may have high CA-125 levels even when the disease has entered remission. Moreover, elevated CA-125 levels and radiological imaging results may precede symptoms of relapse but do not directly detect minimal residual disease or the development of chemoresistance, which poses significant risks to the patient. Therefore, there is a need for faster and more precise diagnostics and, in the future, effective disease monitoring for the emergence of treatment resistance and recurrence.

Although attempts are being made to sequence primary tumors, the utility of this type of material has several limitations: the tumor (not always accessible and often embedded in a paraffin block) is a source of fragmented nucleic acids of poor quality, which ultimately results in unreliable coverage during sequencing. Furthermore, traditional biopsy is prone to significant error due to the heterogeneity of the tumor mass and the inability to obtain a representative sample. All these mentioned issues can be addressed by liquid biopsies.

Currently used biological sources for liquid biopsies exhibit suboptimal sensitivity (too many false-negative results) in cancer diagnostics, especially in patients with localized disease. To date, none of the existing blood-derived elements-such as circulating tumor DNA, exosomes, or circulating tumor cells-has been utilized for multi class cancer diagnostics. This hampers the implementation of liquid biopsies for early cancer detection. On the other hand, molecular RNA analysis of platelets can provide valuable diagnostic information for all cancer patients examined. Importantly, platelet transcriptome analysis also holds potential for other conditions, such as ischemic heart disease, where changes in platelet RNA profiles have also been confirmed.

An alternative to the serum marker CA-125 for ovarian cancer diagnostics could be the use of liquid biopsies, which offer minimally invasive sample collection and surpass traditional primary tumor biopsies currently used in cancer assessment. The most commonly studied material in liquid biopsies is blood, which, in addition to circulating tumor cells and circulating tumor DNA, serves as a source of tumor-educated platelets (TEPs). Using high-throughput sequencing, the transcriptome (RNA) of platelets collected from patients can be profiled. Based on protocols for platelet isolation, RNA extraction, and subsequent sequencing of this material, files containing the platelet RNA profile of individual patients can be generated. These files are then analyzed using a developed artificial intelligence algorithm.

This classification method, used for diagnosing cancer based on liquid biopsies, outperforms currently used solutions in effectiveness. However, the sequencing process itself is complex and relatively expensive, and each use of artificial intelligence methods based on multidimensional sequencing data can be computationally costly. These drawbacks are mitigated by a solution based on a PCR panel. This approach allows for the rapid acquisition of expression profiles for selected transcripts, at a significantly lower cost than traditional sequencing.

In the state-of-the-art, solutions utilizing the liquid biopsy for diagnosing various types of cancer are known.

The Galleri test, developed by the California-based company Grail, is designed to detect more than 50 types of cancer from a blood sample (detecting two out of three cancers in 5,000 individuals) by identifying distinct changes in genetic code fragments of tumor cell DNA that carry specific markers. Additionally, in 85% of cases, it identifies the primary site of cancer development. The test depends on the amount of genetic material present in the bloodstream; therefore, if cancer cells do not release enough mutated genetic material into the bloodstream, the test cannot detect it.
An alternative approach for detecting cancer in liquid biopsy material is presented by the CancerSEEK solution (Cohen et al., Science 2018). This method involves a non-invasive multi-analyte test that simultaneously assesses the presence of mutations and eight protein biomarkers associated with cancer.

There is therefore a demand for new diagnostic methods that enable precise classification of biological material samples from liquid biopsies for ovarian cancer diagnosis, while being fast and sensitive.

The inventors, in their previous research, focused on RNA sequencing-based analyses, which is an expensive and less accessible method [1-6]. To accelerate the diagnostic process and reduce costs, they unexpectedly developed a platform whose uniqueness lies in the careful selection of molecular probes used in plates applied to the RT-qPCR platform.

The objective of the invention is to provide a non-invasive method for analyzing and classifying biological material samples for the detection and diagnosis of ovarian cancer, specifically designed for screening purposes. The process of ovarian cancer detection is characterized by the following stages:
a). assessment of the RNA expression levels of the reference genes: MPP1, GUCY1B3, IQGAP2 and the studied genes: ITGA2B, NFKBIA, OSBP2, GAPDH, RGCC, SMOX, RPL9, RPS5, RPS21, KSR1, RPL37, RPL23A, ADAM10, analysed with respect to the referfence genes. All genes are assayed with the use of the following molecular probes: MPP1-Hs00609971_m1, GUCY1B3-Hs00168336_m1, IQGAP2-Hs01117929_m1, ITGA2B-Hs01116228_m1, NFKBIA-Hs00355671_g1, OSBP2-Hs01066297_m1, GAPDH-Hs99999905_m1, RGCC-Hs00204129_m1, SMOX-Hs00602494_m1, RPL9-Hs01552541_g1, RPS5-Hs00734849_g1, RPS21-Hs00744406_s1, KSR1-Hs01075797_m1, RPL37-Hs03044965_g1, RPL23A-Hs01921329_g1, and ADAM10-Hs00153853_m1, in the sample belonging to a donor, and
b). a comparative analysis of the expression levels of the studied genes measured in step a) with the expression levels of these genes in an individual without ovarian cancer using the comparative ΔΔCT method, whereby the comparison of gene expression levels allows for the determination of a diagnostic index. This index is a numerical value ranging from 0 to 1, which indicates the probability of ovarian cancer presence in the analyzed biological sample.

Advantageously, the method according to the invention is computer-assisted using RT-qPCR probes with the application of an algorithm.

Advantageously, an increase in the classification index to a value of 1 determines a high probability of ovarian cancer occurrence.

Advantageously, a classification index value close to 0 indicates a low probability of ovarian cancer occurrence, whereby only 14% of patients with ovarian cancer exhibited a lower index level than the highest observed among healthy patients.

Advantageously, the method according to the invention demonstrates varied accuracy of 94.12%, with a sensitivity of 88.24% and a specificity of 100%.

Advantageously, the biological material sample is a blood sample.

Advantageously, the method according to the invention allows for the simultaneous assessment of expression levels in at least 8 patients.

The invention also concerns an RT-qPCR probe panel configured to perform the steps of the analysis and classification method according to the invention.

The method according to the invention is characterized by high specificity and sensitivity, enabling the rapid acquisition of expression profiles covering selected transcripts, while simultaneously incurring significantly lower costs compared to traditional sequencing methods. Furthermore, the developed machine learning algorithm allows for reliable, repeatable, and objective assessment of the biological material under study, which is always a challenge when dealing with multidimensional data (thousands of analyzed transcripts).

The method according to the invention surpasses the accuracy of market-available tools used in cancer diagnostics, such as GRAIL, CancerSEEK, and the method previously published by our collaborators: thromboSeq (Best et al., Nature Protocols 2019). It has also achieved comparable accuracy to the imPlatelet method developed by our team (Pastuszak et al., Molecular Oncology 2021), while being characterized by significantly lower complexity, faster operation, and lower cost. Additionally, compared to the latest study on TEPs (in `t Veld et al., Cancer Cells 2022), the method described in the invention achieves higher overall accuracy in ovarian cancer classification despite having lower specificity.
The terms used above and in the description and patent claims have the following meanings:
**Biological material** - refers to blood or a fragment of tissue or tumor.
**Liquid biopsy** - refers to the collection of biological material in a non-solid form.
The subject of the invention is presented using drawing figures, among which:
Fig. 1 presents a schematic of the method according to the invention, in which a sample is collected from a patient suspected of having cancer, the collected material is subjected to quality control and RT-qPCR analysis, after which the obtained readings are normalized, and the developed algorithm classifies the sample as originating from a patient with ovarian cancer or from a patient without cancer.
Fig. 2 presents the distribution of classification results in an independent test set, where:
   (a) represents predictions for patients with ovarian cancer,
   (b) represents results for healthy patients or those with benign lesions.
Fig. 3 presents ROC curves for an independent test set containing 17 patients with ovarian cancer, two healthy volunteers, and three patients with benign pathological lesions.

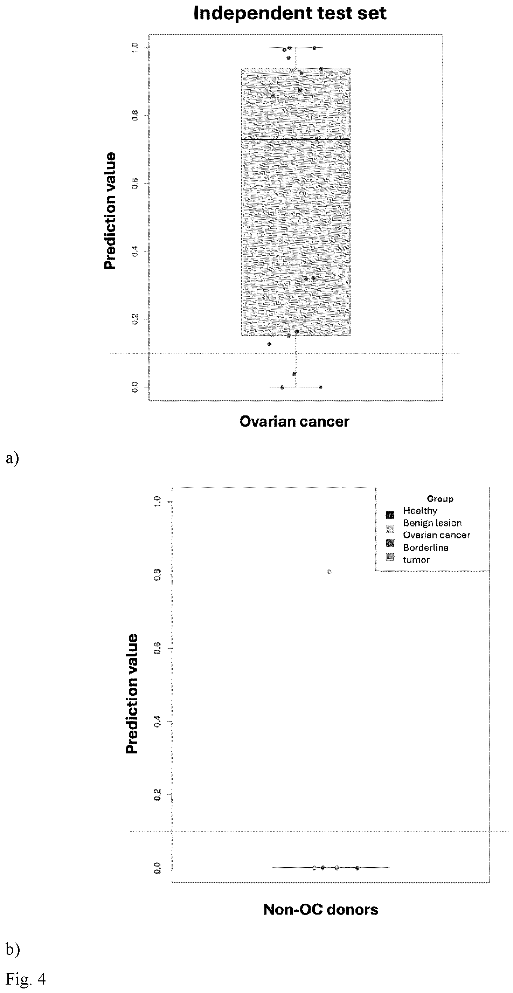

Fig. 4 presents ROC curves for an independent test set limited to healthy patients or those with ovarian cancer.

The invention has been illustrated using the following exemplary embodiments.

### Embodiments

To determine a limited set of transcripts enabling the construction of an RT-qPCR panel, an artificial intelligence algorithm was developed to distinguish between patients with ovarian cancer, healthy donors, and patients with benign gynecological lesions. The analysis included 133 ovarian cancer patients, 32 patients with benign gynecological lesions, and 231 healthy volunteers.

The developed algorithm was analyzed for the significance of individual transcripts in its effectiveness, and a statistical analysis of the expression distributions of the individual transcripts in the studied patient groups was conducted. Based on this, 13 most promising transcripts were identified, enabling high-efficiency classification based on sequencing data.

Additionally, 3 reference genes were identified, exhibiting high expression stability among donors regardless of their assigned category (healthy versus diseased). Based on these findings, a plate for RT-qPCR was developed, allowing the detection of patients with ovarian cancer.

Based on the analysis of the proprietary solution developed using sequencing of plates, a probe panel was designed to assess the expression of 3 reference genes for normalizing the obtained data and 13 genes for classification purposes. The TLDA 384-well plate (TaqMan Low Density Array, Thermo Fisher Scientific) developed according to the invention for RT-qPCR enables the evaluation of the expression levels of the following genes: MPP1, GUCY1B3, IQGAP2 (reference genes) and the studied genes: ITGA2B, GAPDH, NFKBIA, RGCC, OSBP2, SMOX, RPL9, RPS5, RPS21, KSR1, RPL37, RPL23A, ADAM10 analysed with the use of the following molecular probes: MPP1-Hs00609971_m1, GUCY1B3-Hs00168336_m1, IQGAP2-Hs01117929_m1, ITGA2B-Hs01116228_m1, NFKBIA-Hs00355671_g1, OSBP2-Hs01066297_m1, GAPDH-Hs99999905_m1, RGCC-Hs00204129_m1, SMOX-Hs00602494_m1, RPL9-Hs01552541_g1, RPS5-Hs00734849_g1, RPS21-Hs00744406_s1, KSR1-Hs01075797_m1, RPL37-Hs03044965_g1, RPL23A-Hs01921329_g1, and ADAM10-Hs00153853_m1.

Using the developed panel, an additional 46 patients were examined: 34 with ovarian cancer, 5 healthy individuals, 6 with benign gynecological pathological changes, and one with a borderline ovarian tumor (BOT), which was treated as malignant in the binary classification. Half of the patients were used to calibrate the algorithm to the new type of data, as RT-qPCR analysis results exhibit different value ranges compared to transcriptome sequencing data. The other half of the patients were used as an independent test set to evaluate the effectiveness of the developed solution. The solution developed under this invention enables the distinction between samples collected from oncology patients and those collected from healthy volunteers. The method according to the invention is based on biological material in the form of platelets (liquid biopsy). This type of biological material can be classified as cancerous, in contrast to a liquid biopsy collected from a healthy volunteer.

After the sample is collected for testing, the following steps are performed: isolation of platelets, RNA isolation from the platelets, reverse transcription, and then quantitative PCR using the developed plate. The files generated during the RT-qPCR process undergo quality control, normalization, and annotation. The identified expression profile of the analyzed transcript panel is then evaluated by a trained classifier (half of the samples were used to calibrate the algorithm to RT-qPCR data.) The trained algorithm can subsequently test further samples and classify them binarily as "cancer" or "sample from a healthy individual."

According to the present invention, the TLDA plate for qPCR, containing a proprietary probe panel, enables accurate assessment of the expression of a selected panel of transcripts. The accompanying classification method allows for highly effective differentiation between patients with ovarian cancer and healthy individuals. The maximum throughput of this method allows for the analysis and classification of biological material collected simultaneously from 8 patients.

### Example 1

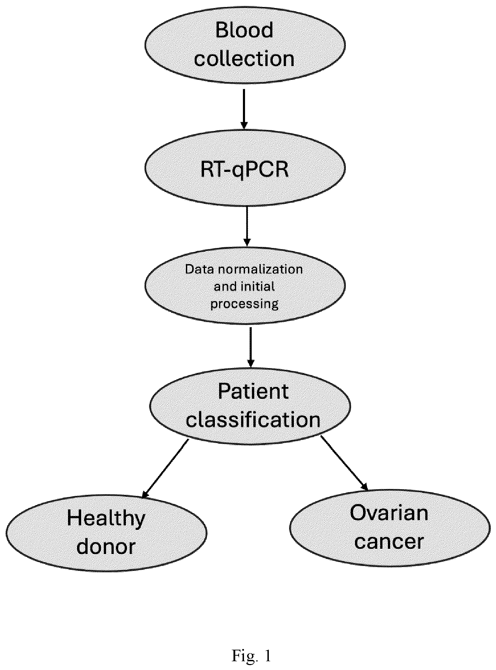

The method according to the invention was developed based on blood platelets, so-called liquid biopsies, collected from patients or healthy volunteers (Fig. 1). Whole blood was collected into 6-ml EDTA Vacutainer tubes, after which the tubes were centrifuged to obtain platelet-rich plasma (centrifugation for 20 minutes at 120xg). The plasma was then pelleted at the bottom of an Eppendorf tube (centrifugation for 20 minutes at 360xg) and subsequently resuspended carefully in RNAlater buffer (Thermo Scientific). After overnight incubation of the samples at 4°C, the platelets were stored at -80°C, and RNA was extracted using the RNeasy isolation kit (Qiagen). The quality of the isolated nucleic acid was verified using the RNA 6000 Picochip (Agilent). High-quality RNA, with clear 5S, 18S, and 28S ribosomal RNA peaks and/or an RIN value > 7, was selected for cDNA synthesis using the SuperScript II kit (Thermo Fisher Scientific). RT-qPCR was then performed using customized probes applied to a TaqMan TLDA plate (Thermo Fisher Scientific). The developed classification algorithm was used for data analysis, with the algorithm being closed and not subject to further training.

After performing RT-qPCR-based experiments in the laboratory, a comparative analysis of the expression levels of the studied genes relative to reference genes is conducted. For this purpose, the comparative ΔΔCT method is used. The ΔΔCT method consists of the following steps. The expression level of each gene for each sample is determined in three replicates. Based on these replicates, the mean value is calculated. The difference between the expression levels of the studied genes and the expression levels of the reference genes (ΔCT) is determined. The calculated difference can then be compared to the differences obtained for a reference sample (healthy). Ultimately, the fold expression of each gene is obtained. For example, an expression result for the KSR1 gene equal to 2 indicates that the expression of this specific gene is twice as high as that of the reference sample.

The obtained files undergo quality control. Patients for whom there is high variability in expression levels across technical replicates are excluded from the analysis. The numerical data are then normalized using the double-delta method. Each patient is represented by a vector of 16 numbers, where each number corresponds to the normalized expression level of one transcript. This vector is then passed to the classification algorithm, which provides the proposed diagnosis as the output.

### Effectiveness of the method according to the Invention

The presented method, based on the RT-qPCR panel, was subsequently tested on an independent test set. The solution achieved a balanced accuracy of 84%, with sensitivity and specificity equal to 88.24% and 80%, respectively (Table 1).

**Table 1. Classification Effectiveness of the RT-qPCR Panel-Based Solution According to the Invention. The classification results are presented for an independent test set, including patients with benign lesions. The outcome is shown for binary classification based on the defined decision threshold.**

| | **Independent test set** |
|---|---|
| | **Result** |
| **Sensitivity** | 88.24% |
| **Specificity** | 80% |
| **Balanced accuracy** | 84.12% |
| **AUC** | 81.18% |

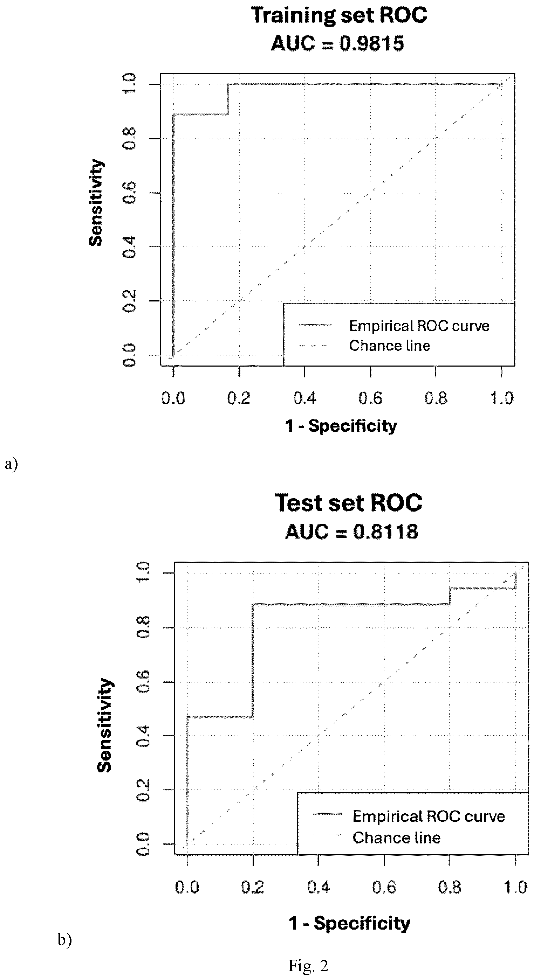

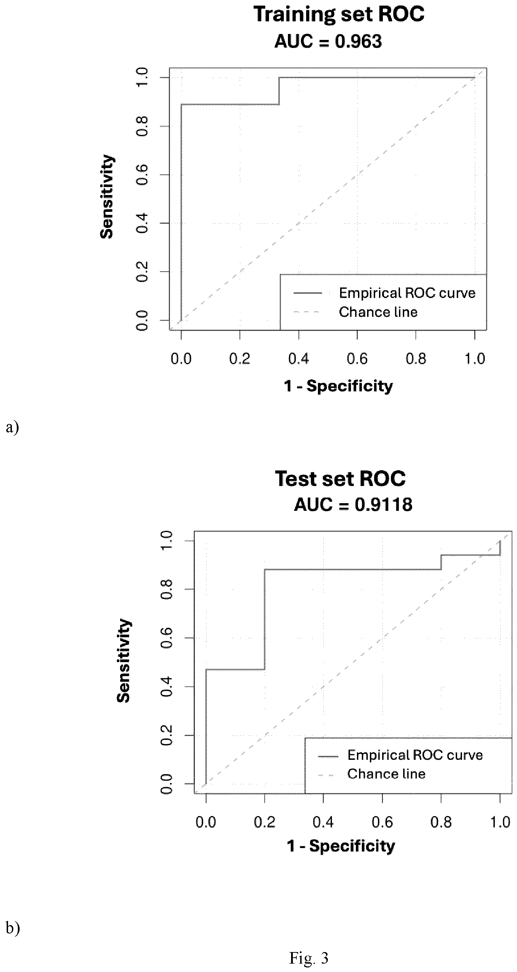

The generated ROC curves demonstrated high performance, with an AUC (Area Under Curve) value of 81.18% in the independent dataset (Fig. 2). When patients with benign pathological lesions were excluded from the test set, the balanced accuracy increased to 94.12%, with sensitivity of 88.24% and specificity of 100% (Table 2). The AUC of the ROC curve increased to 91.18% (Fig. 3).

**Table 2. Classification Effectiveness of the RT-qPCR Panel-Based Solution According to the Invention. The classification results are presented for an independent test set, excluding patients with benign pathological lesions. The outcome is shown for binary classification based on the defined decision threshold.**

| | **Independent test set** |
|---|---|
| | **Result** |
| **Sensitivity** | 88.24% |
| **Specificity** | 100% |
| **Balanced accuracy** | 94.12% |
| **AUC** | 91.18% |

To gain a deeper understanding of the classification dynamics for ovarian cancer patients, individuals with benign lesions, and healthy volunteers, a ranking of scores (the so-called TEPs score) was generated for samples in the test set (Fig. 2). The TEPs score represents non-binary classifier outputs that, after applying an appropriate threshold, can yield a final classification. A TEPs score of 0 indicates a healthy donor, while a score close to 1 indicates a cancer case. The value is an intermediate measure of the probability that a sample is cancerous. While healthy donors were always correctly classified, there was significant overlap between ovarian cancer samples and benign controls, regardless of the disease stage.

### References:

1. Cygert S, Pastuszak K, Górski F, Sieczczyński M, Juszczyk P, Rutkowski A, Lewalski S, Różański R, Jopek MA, Jassem J, Czyzewski A, Wurdinger T, Best MG, Zaczek AJ, Supernat A. Platelet-Based Liquid Biopsies through the Lens of Machine Learning. Cancers (Basel). 2023 Apr 17;15(8):2336. doi:10.3390/cancers15082336. PMID: 37190262; PMCID: PMC10136732.
2. Gao Y, Liu CJ, Li HY, Xiong XM, Li GL, In't Veld SGJG, Cai GY, Xie GY, Zeng SQ, Wu Y, Chi JH, Liu JH, Zhang Q, Jiao XF, Shi LL, Lu WR, Lv WG, Yang XS, Piek JMJ, de Kroon CD, Lok CAR, Supernat A, 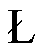 apińska-Szumczyk S, 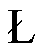 ojkowska A, Zaczek AJ, Jassem J, Tannous BA, Sol N, Post E, Best MG, Kong BH, Xie X, Ma D, Wurdinger T, Guo AY, Gao QL. Platelet RNA enables accurate detection of ovarian cancer: an intercontinental, biomarker identification study. Protein Cell. 2023 Jun 7;14(6):579-590. doi: 10.1093/procel/pwac056. PMID: 36905391; PMCID: PMC 10246718.
3. In 't Veld SGJG, Arkani M, Post E, Antunes-Ferreira M, D'Ambrosi S, Vessies DCL, VermuntL, Vancura A, Muller M, Niemeij er AN, Tannous J, MeijerLL, Le Large TYS, Mantini G, Wondergem NE, Heinhuis KM, van Wilpe S, Smits AJ, Drees EEE, Roos E, Leurs CE, Tjon Kon Fat LA, van der Lelij EJ, Dwarshuis G, Kamphuis MJ, Visser LE, Harting R, Gregory A, Schweiger MW, Wedekind LE, Ramaker J, Zwaan K, Verschueren H, Bahce I, de Langen AJ, Smit EF, van den Heuvel MM, Hartemink KJ, Kuijpers MJE, Oude Egbrink MGA, Griffioen AW, Rossel R, Hiltermann TJN, Lee-Lewandrowski E, Lewandrowski KB, De Witt Hamer PC, Kouwenhoven M, Reijneveld JC, Leenders WPJ, Hoeben A, Verdonck-de Leeuw IM, Leemans CR, Baatenburg de Jong RJ, Terhaard CHJ, Takes RP, Langendijk JA, de Jager SC, Kraaijeveld AO, Pasterkamp G, Smits M, Schalken JA, 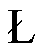 apińska-Szumczyk S, Lojkowska A, Zaczek AJ, Lokhorst H, van de Donk NWCJ, Nijhof I, Prins HJ, Zijlstra JM, Idema S, Baayen JC, Teunissen CE, Killestein J, Besselink MG, Brammen L, Bachleitner-Hofmann T, Mateen F, Plukker JTM, Heger M, de Mast Q, Lisman T, Pegtel DM, Bogaard HJ, Jassem J, Supernat A, Mehra N, Gerritsen W, de Kroon CD, Lok CAR, Piek JMJ, Steeghs N, van Houdt WJ, Brakenhoff RH, Sonke GS, Verheul HM, Giovannetti E, Kazemier G, Sabrkhany S, Schuuring E, Sistermans EA, Wolthuis R, Meijers- Heijboer H, Dorsman J, Oudejans C, Ylstra B, Westerman BA, van den Broek D, Koppers-Lalic D, Wesseling P, Nilsson RJA, Vandertop WP, Noske DP, Tannous BA, Sol N, Best MG, Wurdinger T. Detection and localization of early- and late-stage cancers using platelet RNA. Cancer Cell. 2022 Sep 12;40(9):999-1009.e6. doi: 10.1016/j.ccell.2022.08.006. Epub 2022 Sep 1. PMID: 36055228.
4. Lukasiewicz M, Pastuszak K, 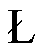 apińska-Szumczyk S, Różański R, Veld SGJGI', Bieńkowski M, Stokowy T, Ratajska M, Best MG, Würdinger T, Zaczek AJ, Supernat A, Jassem J. Diagnostic Accuracy of Liquid Biopsy in Endometrial Cancer. Cancers (Basel). 2021 Nov 16;13(22):5731. doi: 10.3390/cancers13225731. PMID: 34830891; PMCID: PMC8616122.
5. Supernat A, Pop 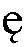 da M, Pastuszak K, Best MG, Grešner P, Veld SI', Siek B, Bednarz-Knoll N, Rondina MT, Stokowy T, Wurdinger T, Jassem J, Zaczek AJ. Transcriptomic landscape of blood platelets in healthy donors. Sci Rep. 2021 Aug 3;11(1):15679. doi: 10.1038/s41598-021-94003-z. PMID: 34344933; PMCID: PMC8333095.
6. Pastuszak K, Supernat A, Best MG, In't Veld SGJG, 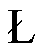 apińska-Szumczyk S, Lojkowska A, Różański R, Zaczek AJ, Jassem J, Würdinger T, Stokowy T. imPlatelet classifier: image-converted RNA biomarker profiles enable blood-based cancer diagnostics. Mol Oncol. 2021 Oct;15(10):2688-2701.doi: 10.1002/1878-0261.13014. Epub 2021 Jun 20. PMID: 34013585; PMCID: PMC8486571.

## Claims

1. A method for analyzing and classifying a sample of biological material to detect ovarian cancer in an individual using the RT-qPCR method, **characterized in that** it comprise the following steps:
a) analysis of the expression level of the reference genes: MPP1, GUCY1B3, IQGAP2 and the expression of the analysed genes: ITGA2B, NFKBIA, OSBP2, GAPDH, RGCC, SMOX, RPL9, RPS5, RPS21, KSR1, RPL37, RPL23A, ADAM10 with respect to the reference genes and with the use of the following molecular probes: MPP1-Hs00609971_m1, GUCY1B3-Hs00168336_m1, IQGAP2-Hs01117929_m1, ITGA2B-Hs01116228_m1, NFKBIA-Hs00355671_g1, OSBP2-Hs01066297_m1, GAPDH-Hs99999905_m1, RGCC-Hs00204129_m1, SMOX-Hs00602494_m1, RPL9-Hs01552541_g1, RPSS-Hs00734849_g1, RPS21-Hs00744406_s1, KSR1-Hs01075797_m1, RPL37-Hs03044965_g1, RPL23A-Hs01921329_g1, and ADAM10-Hs00153853_m1, in a donor's sample, and
b) a comparative analysis of the expression levels of the genes examined in step a) with the expression levels of these genes in an individual without ovarian cancer, performed using the comparative ΔΔCT method, wherein the comparison of gene expression levels enables the determination of a diagnostic index, represented as a numerical value ranging from 0 to 1, which indicates the probability of ovarian cancer presence in the analyzed biological sample.

2. The method according to claim 1, **characterized in that** it is computer-assisted using RT-qPCR probes and an algorithm.

3. The method according to claim 1, **characterized in that** an increase in the classification index to a value of 1 indicates a high probability of ovarian cancer occurrence.

4. The method according to claim 1, **characterized in that** a classification index value close to 0 indicates a low probability of ovarian cancer occurrence.

5. The method according to claim 1, **characterized in that** it demonstrates a varied accuracy of 94.12%, with 88.24% sensitivity and 100% specificity.

6. The method according to claim 1, **characterized in that** the biological material sample is a blood sample.

7. The method according to claim 1, **characterized in that** it allows for the simultaneous assessment of expression levels in at least 8 patients.

8. An RT-qPCR probe panel, configured to perform the steps of the analysis and classification method as specified in any of claims 1 to 7.
